# EUROPEAN PATENT APPLICATION

(11) **EP 3 088 388 A1**
(43) Date of publication of application: **02.11.2016**
(21) Application number: 15165912.5
(22) Date of filing: 30.04.2015
(51) Int. Cl.: C07C 405/00, C07D 493/04, A61K 31/5575, A61P 27/06

(54) **NITRIC OXIDE DONATING DERIVATIVES OF PROSTAGLANDINS**

(71) Applicant: Nicox S.A., 06560 Sophia Antipolis - Valbonne (FR)
(72) Inventor: Almirante, Nicoletta, 20155 MILANO (IT); Storoni, Laura, 20031 CESANO MADERNO (MB) (IT); Bastia, Elena, 20100 MILANO (IT); Brambilla, Stefania, 22046 MERONE (CO) (IT); Impagnatiello, Francesco, 20131 MILANO (IT)
(74) Representative: Bianchetti Bracco Minoja S.r.l.

(57) **Abstract**

The present invention relates to nitrooxyderivatives of tafluprost acid, their use for the treatment of glaucoma and ocular hypertension and formulation containing nitrooxyderivatives of tafluprost acid.

## Description

### Field of the invention

The present invention relates to nitrooxy-esters of tafluprost acid, their use for the treatment of glaucoma and ocular hypertension and formulations containing nitrooxy-esters of tafluprost acid.

### Background of the invention

Glaucoma is a group of eye disorders leading to progressive damage to the optic nerve, and is characterized by loss of nerve tissue resulting in loss of vision. The most common form of glaucoma, primary open-angle glaucoma, is associated with an increase in the fluid pressure inside the eye. This increase in pressure may cause progressive damage to the optic nerve and loss of nerve fibers. Advanced glaucoma may even cause blindness. Glaucoma is the second leading cause of blindness in the U.S. It most often occurs in people over age 40, although a congenital or infantile form of glaucoma exists.

There are many types of glaucoma. The most common form of glaucoma, primary open-angle glaucoma, develops slowly and usually without any symptoms. It initially affects peripheral or side vision, but can advance to central vision loss. If left untreated, glaucoma can lead to significant loss of vision in both eyes, and may even lead to blindness.

A less common type of glaucoma, acute angle closure glaucoma, usually occurs abruptly due to a rapid increase of pressure in the eye.

Secondary glaucoma occurs as a result of an injury or other eye disease. It may be caused by a variety of medical conditions, physical injuries, and eye abnormalities. Infrequently, eye surgery can be associated with secondary glaucoma.

Normal-tension glaucoma, also known as low-tension glaucoma, is characterized by progressive optic nerve damage and visual field loss with normal intra ocular pressure (IOP) and may account for as many as one-third of the cases of open-angle glaucoma in U.S. Normal-tension glaucoma is thought to be, in part, due to poor blood flow to the optic nerve, which leads to death of the ganglion cells which carry impulses from the retina to the brain. A pressure lower than normal is necessary to prevent further visual loss.

The most common first line treatment of glaucoma is drug treatment. Several classes of drugs acting by different mechanisms are used as topically administered ocular therapy to lower IOP. These include beta adrenergic blockers (e.g., timolol), topical carbonic anhydrase inhibitors (e.g., dorzolamide), and alpha 2-adrenergic receptor agonists (e.g., brimonidine), all of which act primarily by decreasing the formation of aqueous humor within the eye. Pilocarpine and epinephrine are clinical agents that also lower IOP in glaucomatous eyes, but these drugs act principally by decreasing the resistance in the trabecular meshwork outflow channels. A third mechanism for lowering IOP in the primate eye is by increasing the outflow of aqueous humor via the uveoscleral route.

Prostaglandin analogs have met an increasing interest for glaucoma therapy as IOP-lowering substances which act primarily by increasing the uveoscleral outflow.

Recently, nitric oxide (NO)-donating prostaglandin derivatives have been studied as IOP-lowering compounds for the treatment of glaucoma and there are some reports on the studies. For example Journal of Ocular Pharmacology and Therapeutics (2010), 26(2), 125-131, Experimental Eye Research (2011), 93(3), 243-249 and Experimental Eye Research (2011), 93(3), 250-255 disclose the IOP lower effect of two NO donating latanoprost acid derivatives. The compound known as BOL-303259-X is now in clinical development for the treatment of primary open-angle glaucoma.

The drug therapies for glaucoma are sometimes associated with significant side effects, for example Timolol and other topically applied beta blockers have been associated with asthma exacerbation, worsening congestive heart failure and, rarely heart block.

Pilocarpine may cause systemic cholinergic effects such as nausea, vomiting, sweating and cutaneous vasodilatation. PGF_{2α} and its esters are characterized by the occurrence of ocular side effects, primarily conjunctival hyperemia.

Patients with glaucoma need to continue treatment for the rest of their lives. Because the disease can progress, only by keeping eye pressure under control can continued damage to the optic nerve and continued loss of visual field be slowed or stopped.

Intraocular pressure is the primary risk factor for glaucoma and lowering IOP to prevent optic nerve injury is the only proven effective treatment. (Kass MA, et al., Arch Ophthalmol, 2002, 120:701-703; The AGIS Investigators, Am J Ophthalmol, 2000, 130:429- 440).

Tafluprost (1-methylethyl (5Z)-7-{(1R,2R,3R,5S)-2-[(1E)-3,3-difluoro-4-phenoxy-1-butenyl]-3,5-dihydroxycyclopentyl}-5-heptenoate) is a prostaglandin analog used to control the progression of glaucoma and to manage ocular hypertension.

EP 0 850 962 discloses the preparation of 15,15-difluoro-15-deoxy PGF_{2α} and its derivatives and their effect in lowering intraocular pressure.

PCT publications WO 2005/068421, WO 2009/136281, WO 2007/000641 and WO 2007/0642 describe (NO)-donating prostaglandin derivatives with increased ocular hypotensive activity. WO 2007/0642 discloses nitrooxy-esters and nitrooxy-amide of tafluprost free acid form.

In spite of all the treatments evolved over decades as described above, there is still a need for new drugs for treatments and therapies for glaucoma and elevated intra ocular pressure.

Thus, this invention provides nitrooxy-esters of tafluprost acid useful for the treatment of glaucoma and elevated intraocular pressure.

### Description of the invention

It has now been found that nitrooxy-esters of tafluprost acid are efficacious and potent ocular hypotensive agents and therefore the nitrooxy-esters of tafluprost acid of the present invention can be employed for treating ocular hypertension and glaucoma, in particular chronic open-angle glaucoma.

An embodiment of the invention relates to compounds of formula (I) 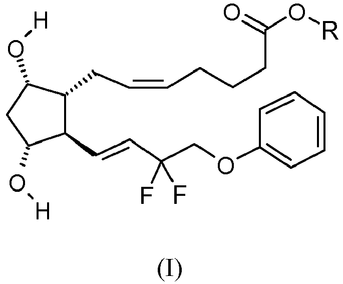 wherein
R is selected from
A1): -CH₂-(CHR¹)-NH-(C=O)-(CH₂)ₘ-[O-(CH₂)ₙ]ₚ-(CH-ONO₂)_{q}-CH₂-ONO₂
A2): -CH₂-(CHR¹)-NH-(C=O)-O-(CH₂)ₘ₁-[O-(CH₂)ₙ]ₚ-(CH-ONO₂)_{q}-CH₂-ONO₂
A3): 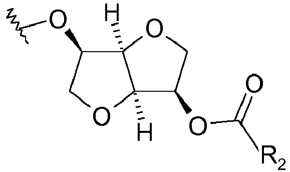 wherein
   R¹ is -H or -CH₃;
   R₂ is -(CH₂)ₘ-[O-(CH₂)ₙ]ₚ-(CH-ONO₂)_{q}-CH₂-ONO₂
   p is 1 or 0,
   q is 1 or 0,
   m is an integer ranging from 1 to 10; preferably m is from 1 to 6;
   m1 is an integer ranging from 2 to 10; preferably m is from 2 to 6;
   n is an integer ranging from 1 to 6; preferably n is 1 or 2.

Preferred linkers having structure A1 are reported below:

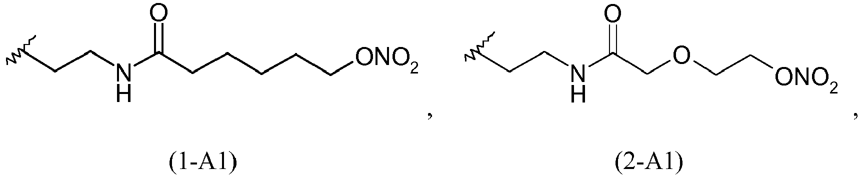

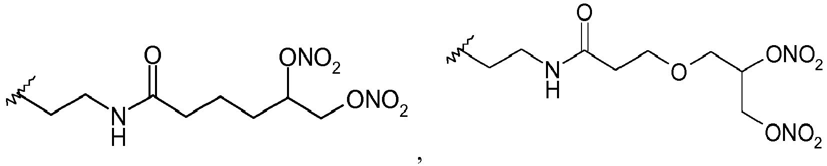

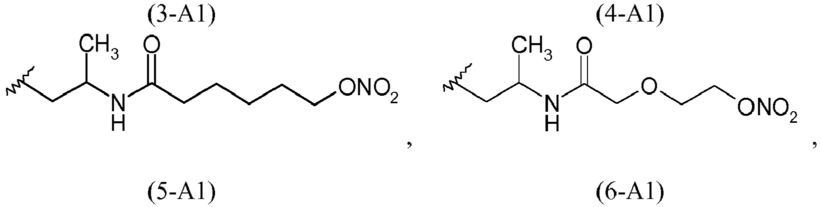

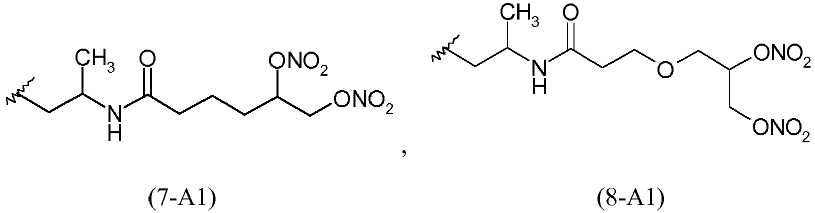

Preferred linkers having structure A2 are reported below:

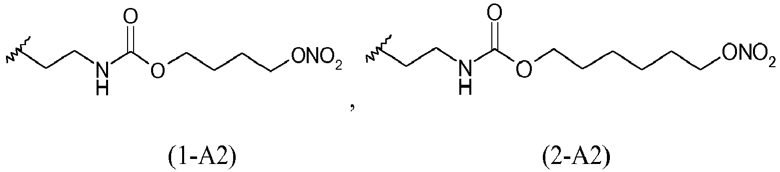

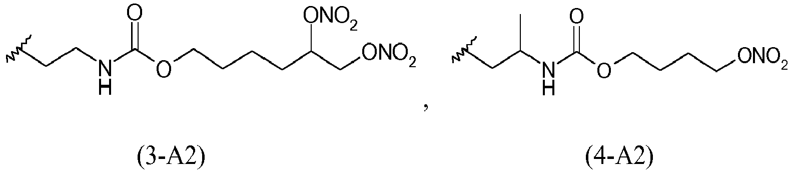

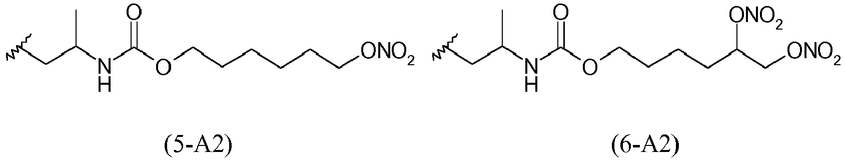

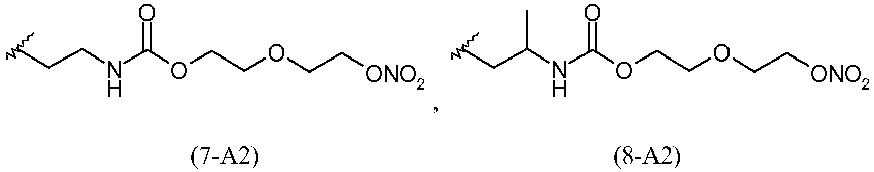

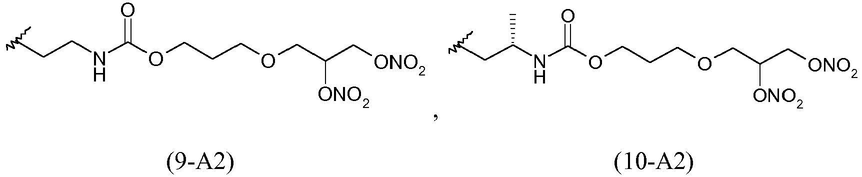

Preferred linkers having structure of the group A3 are reported below:

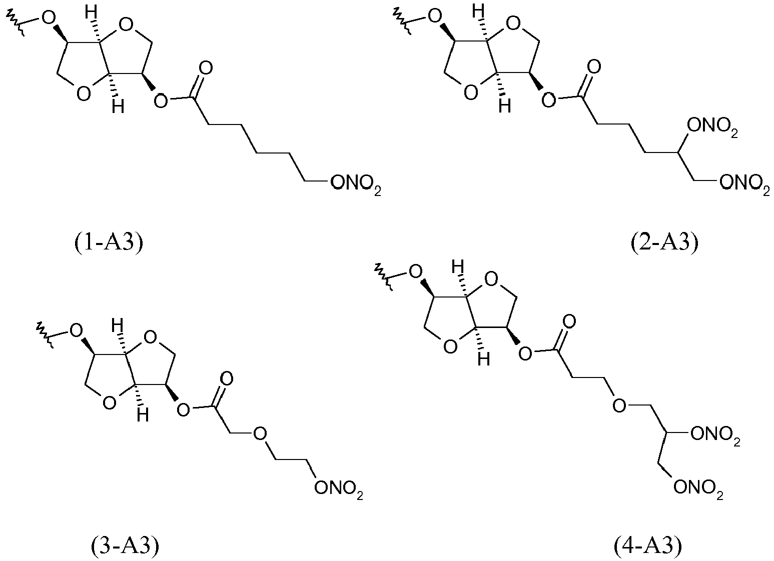

Another embodiment of the invention relates to compounds of formula (I) as defined above wherein
R is A1): -CH₂-(CHR¹)-NH-(C=O)-(CH₂)ₘ-[O-(CH₂)ₙ]ₚ-(CH-ONO₂)_{q}-CH₂-ONO₂
wherein
R₁ is -H or -CH₃;
p is 1 or 0,
q is 1 or 0,
m is an integer ranging from 1 to 10; preferably m is from 1 to 6;
n is an integer ranging from 1 to 6; preferably n is 1 or 2.

Another embodiment of the invention relates to compounds of formula (I) as defined above wherein R is selected from the following group of linkers having structure A1:

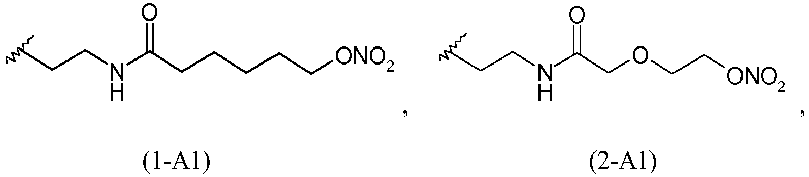

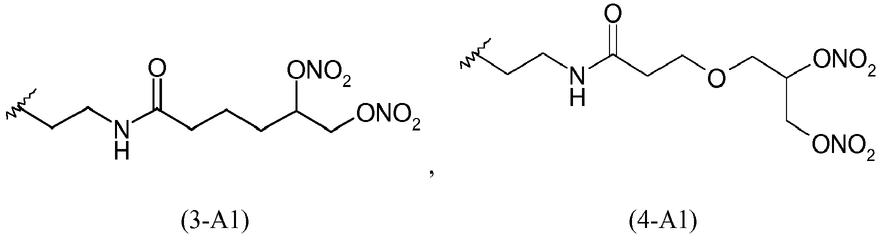

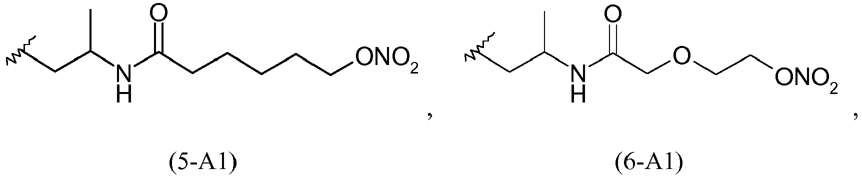

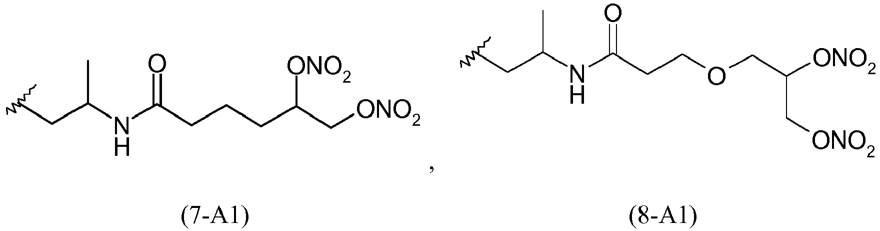

Another embodiment of the invention relates to compounds of formula (I) as defined above or a salt thereof wherein
R is
A2): -CH₂-(CHR¹)-NH-(C=O)-O-(CH₂)ₘ₁-[O-(CH₂)ₙ]ₚ-(CH-ONO₂)_{q}-CH₂-ONO₂
R¹ is -H or -CH₃;
p is 1 or 0,
q is 1 or 0,
m1 is an integer ranging from 2 to 10; preferably m is from 2 to 6;
n is an integer ranging from 1 to 6; preferably n is 1 or 2.

Another embodiment of the invention relates to compounds of formula (I) as defined above wherein R is selected from the following group of linkers having structure A2:

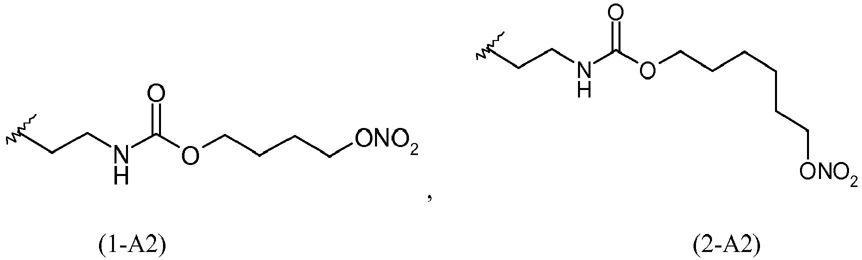

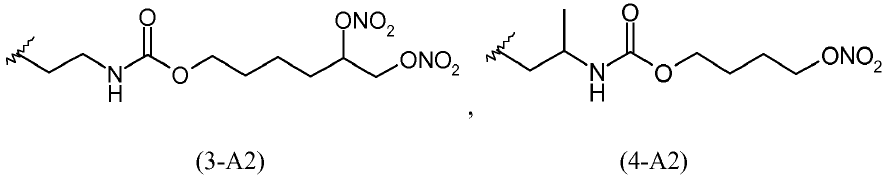

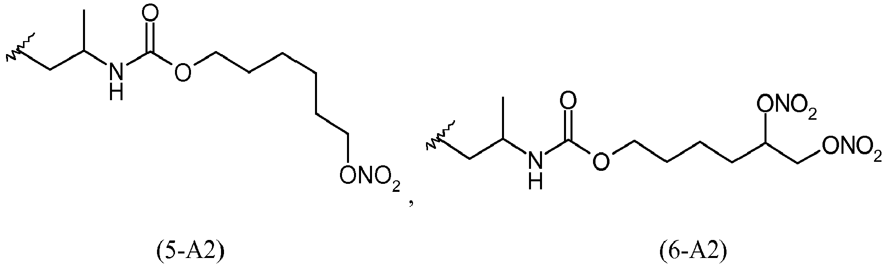

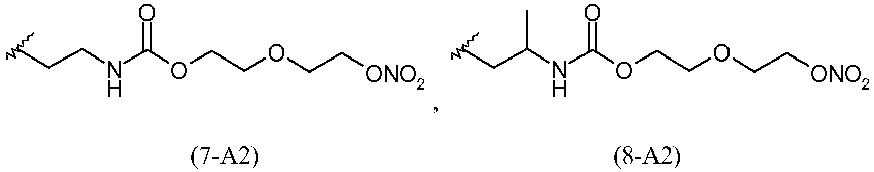

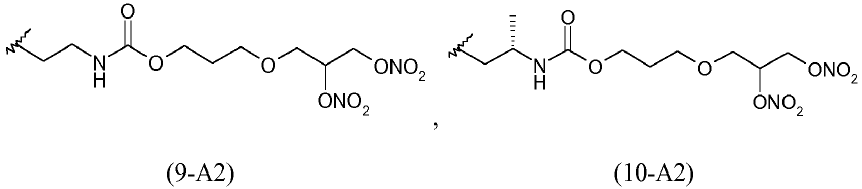

Another embodiment of the invention relates to compounds of formula (I) as defined above wherein
R is A3): 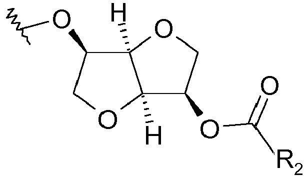 wherein
R₂ is -(CH₂)ₘ-[O-(CH₂)ₙ]ₚ-(CH-ONO₂)_{q}-CH₂-ONO₂
p is 1 or 0,
q is 1 or 0,
m is an integer ranging from 1 to 10; preferably m is from 1 to 6;
n is an integer ranging from 1 to 6; preferably n is 1 or 2.

Another embodiment of the invention relates to compounds of formula (I) as defined above or a salt thereof wherein R is selected from the following group of linkers having structure A3:

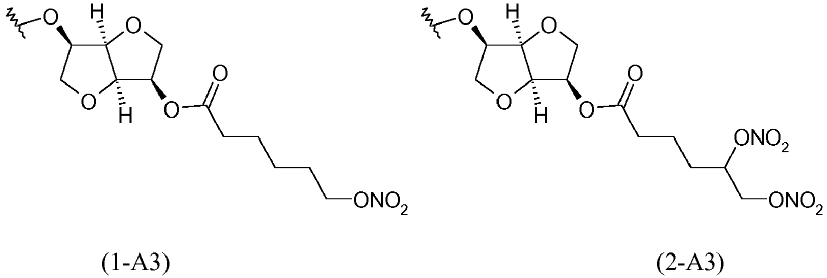

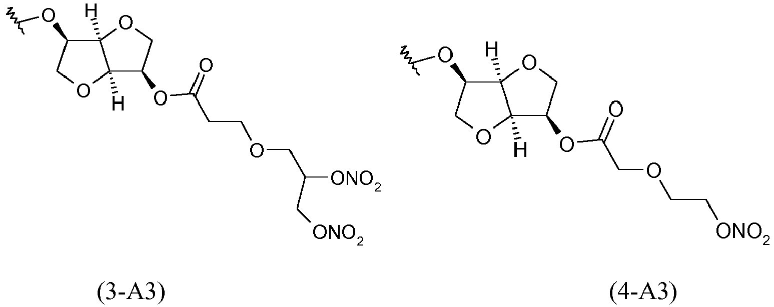

Another embodiment of the inventions relates to a compound of formula (I) selected from the following group of compounds:
(Z)-2-(6-(nitrooxy)hexanamido)ethyl 7-((1R,2R,3R,5S)-2-((E)-3,3-difluoro-4-phenoxybut-1-enyl)-3,5-dihydroxycyclopentyl)hept-5-enoate (Compound (1)) 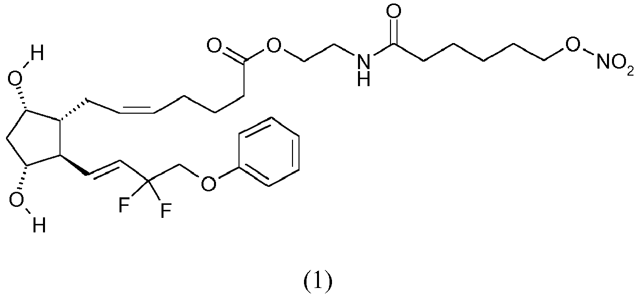
(Z)-2-(5,6-bis(nitrooxy)hexanamido)ethyl 7-((1R,2R,3R,5S)-2-((E)-3,3-difluoro-4-phenoxybut-1-enyl)-3,5-dihydroxycyclopentyl)hept-5-enoate (Compound (2)) 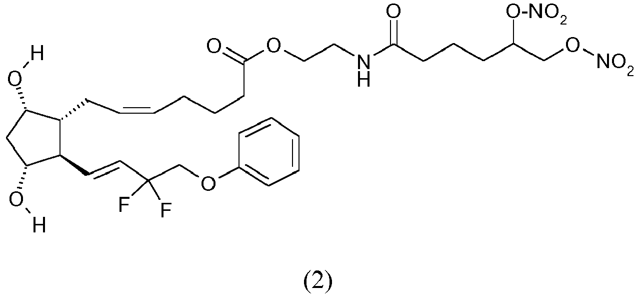
(Z)-2-(2-(2-(nitrooxy)ethoxy)acetamido)ethyl 7-((1R,2R,3R,5S)-2-((E)-3,3-difluoro-4-phenoxybut-1-enyl)-3,5-dihydroxycyclopentyl)hept-5-enoate (Compound (3)) 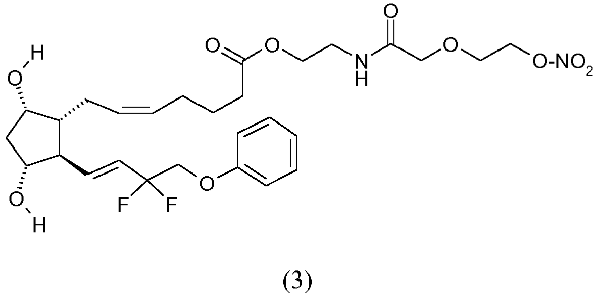
(Z)-2-(3-(2,3-bis(nitrooxy)propoxy)propanamido)ethyl 7-((1R,2R,3R,5S)-2-((E)-3,3-difluoro-4-phenoxybut-1-enyl)-3,5-dihydroxycyclopentyl)hept-5-enoate (Compound (4)) 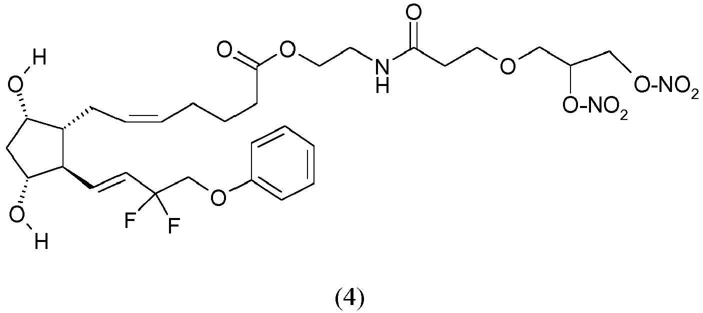
(Z)-((S)-2-(6-(nitrooxy)hexanamido)propyl) 7-((1R,2R,3R,5S)-2-((E)-3,3-difluoro-4-phenoxybut-1-enyl)-3,5-dihydroxycyclopentyl)hept-5-enoate (Compound (5)) 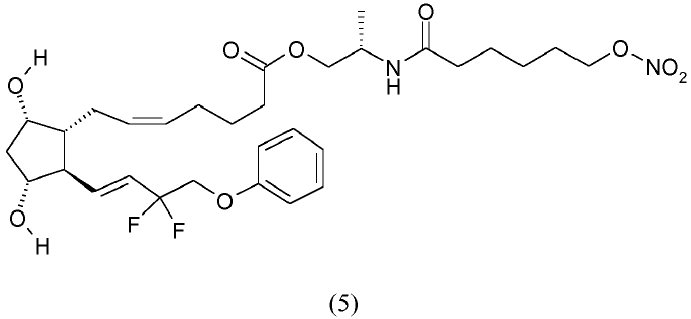
(Z)-((2S)-2-(5,6-bis(nitrooxy)hexanamido)propyl) 7-((1R,2R,3R,5S)-2-((E)-3,3-difluoro-4-phenoxybut-1-enyl)-3,5-dihydroxycyclopentyl)hept-5-enoate (Compound (6)) 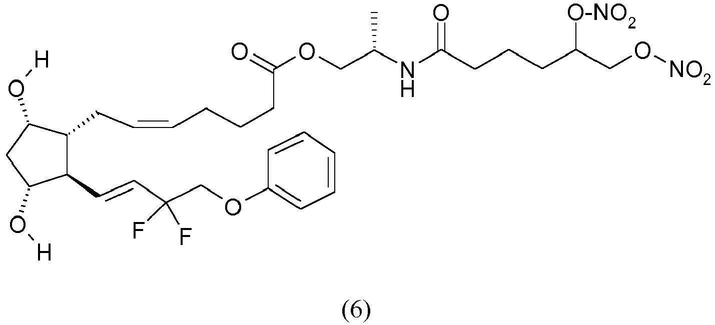
(Z)-((S)-2-(2-(2-(nitrooxy)ethoxy)acetamido)propyl) 7-((1R,2R,3R,5S)-2-((E)-3,3-difluoro-4-phenoxybut-1-enyl)-3,5-dihydroxycyclopentyl)hept-5-enoate (compound (7)) 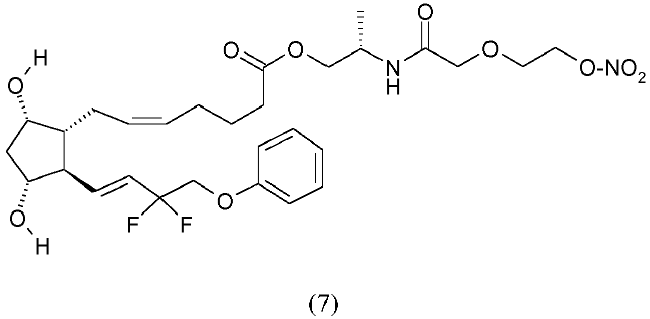
(Z)-((2S)-2-(3-(2,3-bis(nitrooxy)propoxy)propanamido)propyl) 7-((1R,2R,3R,5S)-2-((E)-3,3-difluoro-4-phenoxybut-1-enyl)-3,5-dihydroxycyclopentyl)hept-5-enoate (Compound (8)) 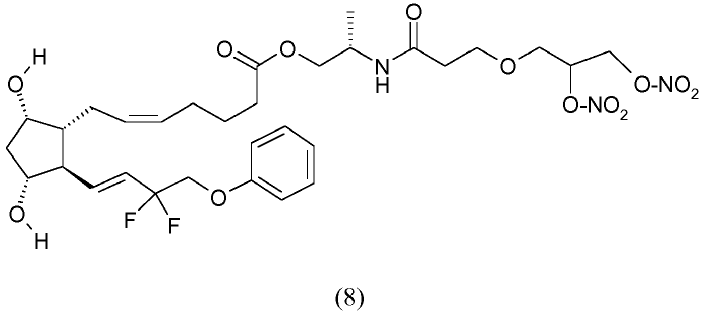
(Z)-2-((4-(nitrooxy)butoxy)carbonylamino)ethyl 7-((1R,2R,3R,5S)-2-((E)-3,3-difluoro-4-phenoxybut-1-enyl)-3,5-dihydroxycyclopentyl)hept-5-enoate (Compound (9)) 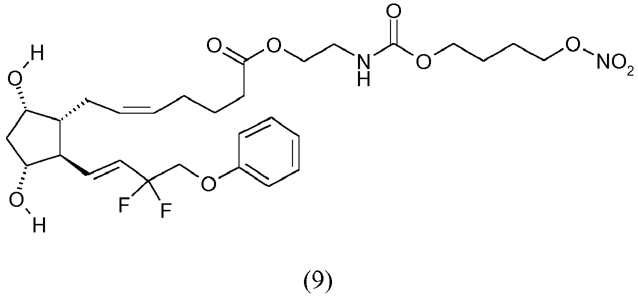
(Z)-((S)-2-((4-(nitrooxy)butoxy)carbonylamino)propyl) 7-((1R,2R,3R,5S)-2-((E)-3,3-difluoro-4-phenoxybut-1-enyl)-3,5-dihydroxycyclopentyl)hept-5-enoate (Compound (10)) 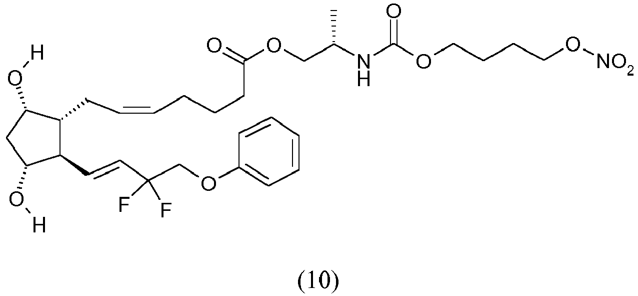
(Z)-2-((6-(nitrooxy)hexyloxy)carbonylamino)ethyl 7-((1R,2R,3R,5S)-21-((E)-3,3-difluoro-4-phenoxybut-1-enyl)-3,5-dihydroxycyclopentyl)hept-5-enoate (Compound (11)) 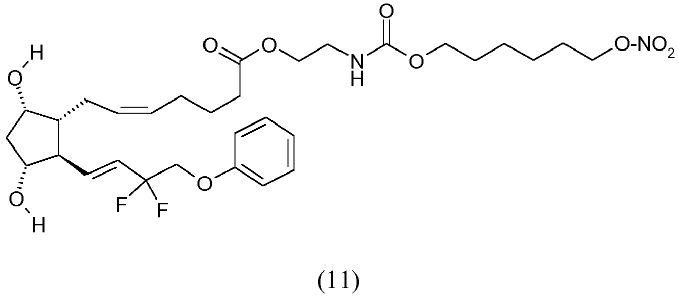
(Z)-((S)-2-((6-(nitrooxy)hexyloxy)carbonylamino)propyl) 7-((1R,2R,3R,5S)-2-((E)-3,3-difluoro-4-phenoxybut-1-enyl)-3,5-dihydroxycyclopentyl)hept-5-enoate (Compound (12)) 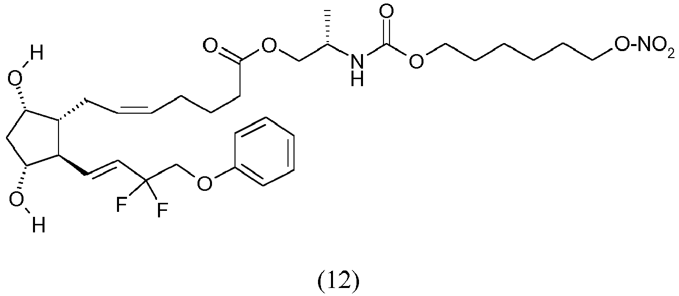
(Z)-2-((5,6-bis(nitrooxy)hexyloxy)carbonylamino)ethyl 7-((1R,2R,3R,5S)-2-((E)-3,3-difluoro-4-phenoxybut-1-enyl)-3,5-dihydroxycyclopentyl)hept-5-enoate (Compound (13)) 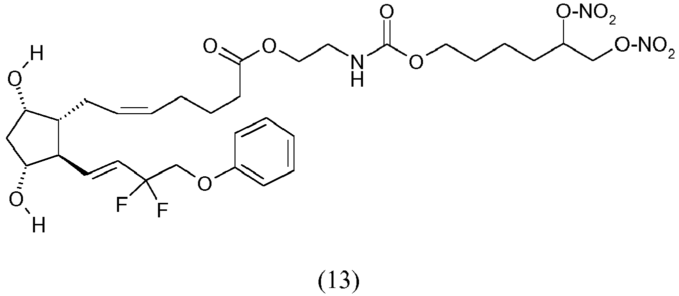
(Z)-((2S)-2-((5,6-bis(nitrooxy)hexyloxy)carbonylamino)propyl) 7-((1R,2R,3R,5S)-2-((E)-3,3-difluoro-4-phenoxybut-1-enyl)-3,5-dihydroxycyclopentyl)hept-5-enoate (Compound (14)) 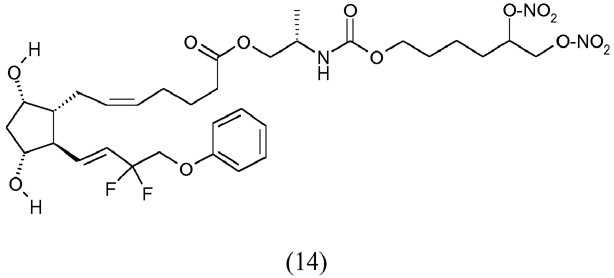
(Z)-2-((2-(2-(nitrooxy)ethoxy)ethoxy)carbonylamino)ethyl 7-((1R,2R,3R,5S)-2-((E)-3,3-difluoro-4-phenoxybut-1-enyl)-3,5-dihydroxycyclopentyl)hept-5-enoate (Compound (15)) 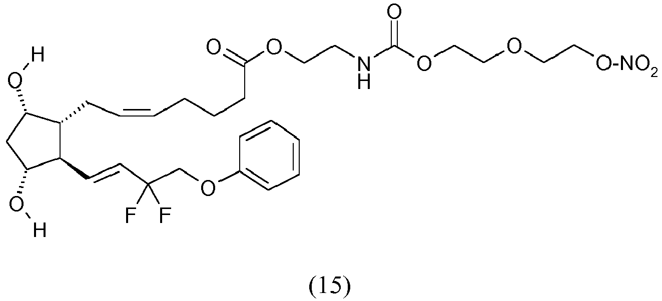
(Z)-((S)-2-((2-(2-(nitrooxy)ethoxy)ethoxy)carbonylamino)propyl) 7-((1R,2R,3R,5S)-2-((E)-3,3-difluoro-4-phenoxybut-1-enyl)-3,5-dihydroxycyclopentyl)hept-5-enoate (Compound (16)) 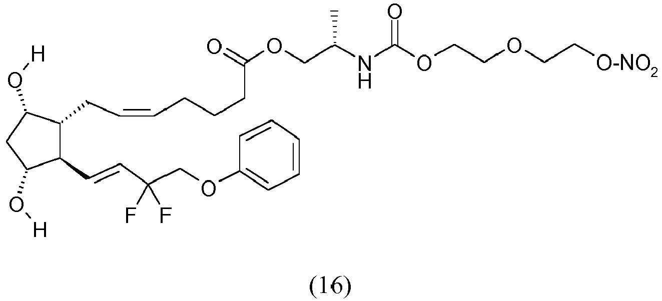
(Z)-2-((3-(2,3-bis(nitrooxy)propoxy)propoxy)carbonylamino)ethyl 7-((1R,2R,3R, 5S)-2-((E)-3,3-difluoro-4-phenoxybut-1-enyl)-3,5-dihydroxycyclopentyl)hept-5-enoate (Compound (17)) 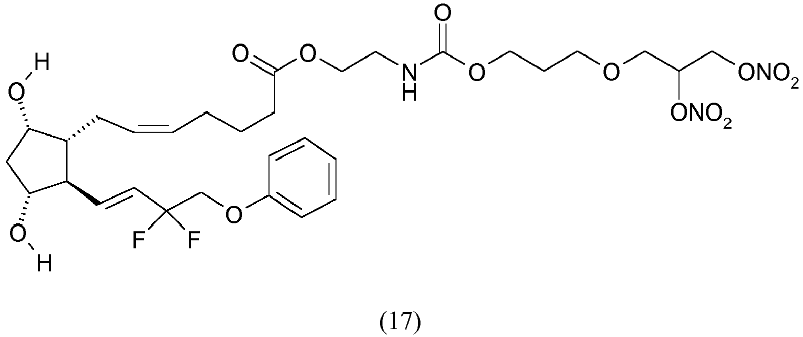
(Z)-((2S)-2-((3-(2,3-bis(nitrooxy)propoxy)propoxy)carbonylamino)propyl) 7-((1R,2R,3R,5S)-2-((E)-3,3-difluoro-4-phenoxybut-1-enyl)-3,5-dihydroxycyclopentyl) hept-5-enoate (Compound (18)) 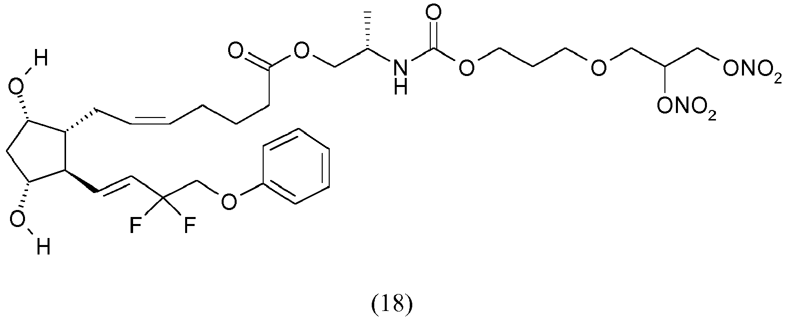
(Z)-((3R,3aR,6R,6aR)-6-(6-(nitrooxy)hexanoyloxy)hexahydrofuro[3,2-b]furan-3-yl) 7-((1R,2R,3R,5S)-2-((E)-3,3-difluoro-4-phenoxybut-1-enyl)-3,5-dihydroxycyclo pentyl)hept-5-enoate (Compound (19)) 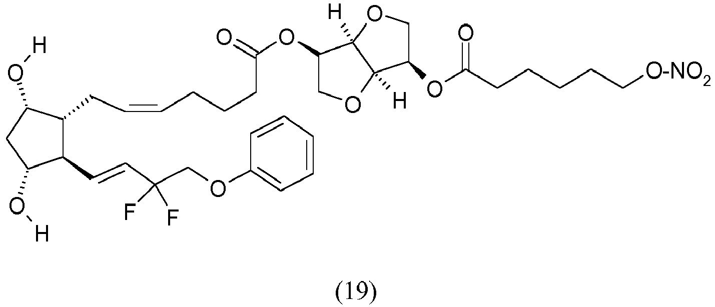
(Z)-((3R,3aR,6R,6aR)-6-(5,6-bis(nitrooxy)hexanoyloxy)hexahydrofuro[3,2-b]furan-3-yl) 7-((1R,2R,3R,5S)-2-((E)-3,3-difluoro-4-phenoxybut-1-enyl)-3,5-dihydroxy cyclopentyl) hept-5-enoate (Compound (20)) 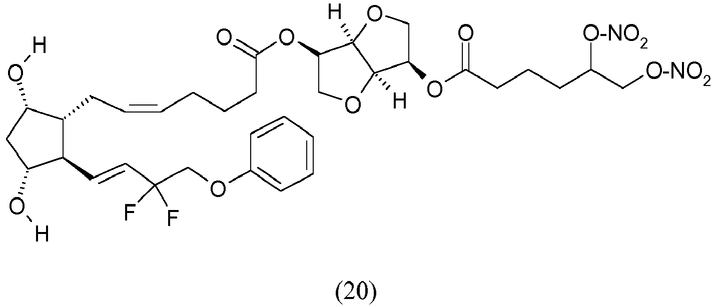
(Z)-((3R,3 aR,6R,6aR)-6-(2-(2-(nitrooxy)ethoxy)acetoxy)hexahydrofuro [3,2-b]furan-3-yl) 7-((1R,2R,3R,5S)-2-((E)-3,3-difluoro-4-phenoxybut-1-enyl)-3,5-dihydroxy cyclopentyl)hept-5-enoate (Compound (21)) 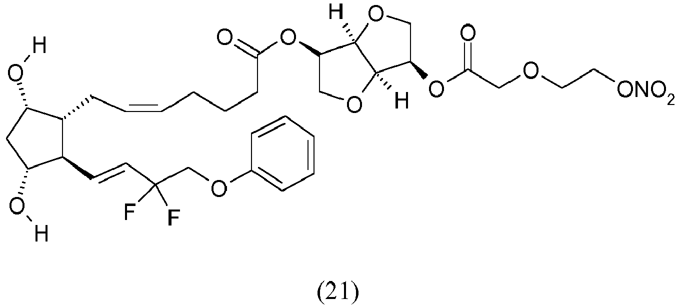
(Z)-((3R,3aR,6R,6aR)-6-(3-(2,3bis(nitrooxy)propoxy)propanoyloxy)hexahydrofuro[3,2-b]furan-3-yl) 7-((1R,2R,3R,5S)-2-((E)-3,3-difluoro-4-phenoxybut-1-enyl)-3,5-dihydroxycyclopentyl)hept-5-enoate (Compound (22))

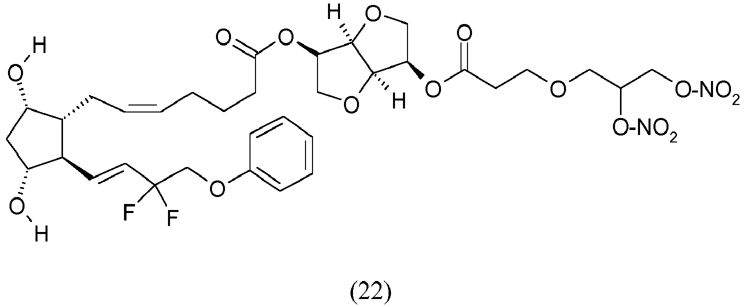

Included within the scope of the present invention are the individual enantiomers of the compounds of formula (I), as well as their racemic and non-racemic mixtures.

Another embodiment provides the use of compound of Formula (I) for treating ocular hypertension.

Another embodiment provides the use of compound of Formula (I) for treating glaucoma in particular primary open angle glaucoma, normal intraocular tension glaucoma, pseudoexfoliation glaucoma, acute angle-closure glaucoma, chronic closed angle glaucoma.

The compound may be provided as part of a pharmaceutical composition as described therein.

In forming the compositions for topical administration, the compounds of the present invention are generally formulated as between about 0.00003 to about 3 percent by weight (wt %) solutions in water at a pH between 4.5 to 8.0. The compounds are preferably formulated as between about 0.0003 to about 1 wt % and, most preferably, between about 0.0015 and about 0.3 wt %.

In another aspect, there is provided a topical ocular pharmaceutical composition. The pharmaceutical composition includes a compound of Formula (I) and a pharmaceutically acceptable excipient. Acceptable excipients may include preservatives, dissolving agents and viscosity agents.

Suitable preservatives include: benzalkonium chloride, thimerosal, chlorobutanol, methyl paraben, propyl paraben, phenylethyl alcohol, edetate disodium, sorbic acid or other agents known to those skilled in the art. Such preservatives are typically employed at a concentration between about 0.001% and about 1.0% by weight. Dissolving agents include: polysorbates for example polyoxyethylene sorbitan monolaurate, and polyoxyethylene sorbitan monooleate for polysorbate 80, polyoxylated castor oil such as polyoxyethylene hydrogenated castor oil 40 and polyoxyethylene hydrogenated castor oil 60, polyoxyl stearate, macrogol, propyleneglycol; or other agents known to those skilled in the art. The dissolving agent may be used solely or in combination with one or more other dissolving agents.

Viscosity agents include, for example, polyvinyl alcohol, polyvinyl pyrrolidone, methyl cellulose, hydroxy propyl methylcellulose, hydroxyethyl cellulose, carboxymethyl cellulose, hydroxy propyl cellulose or other agents known to those skilled in the art.

The ophthalmic composition of the present invention may further contain other additives. Examples of the additives may include osmotic adjusting agents such as sodium chloride, potassium chloride, calcium chloride, sodium bicarbonate, sodium carbonate, magnesium sulfate, sodium hydrogen phosphate, sodium dihydrogen phosphate, dipotassium hydrogen phosphate, borax, sodium hydroxide, hydrochloric acid, isosorbitol, propylene glycol, mannitol, sucrose and glucose; buffering agents such as sodium monohydrogen phosphate and sodium dihydrogen phosphate.

The compound of the present invention can also be used in combination with the following classes of drugs: Beta-adrenergic antagonists including carteolol, levobunolol, metipranolol, timolol hemihydrate; Adrenergic Agonists including non-selective adrenergic agonists such as epinephrine borate, epinephrine hydrochloride, and dipivefrin; and Alpha₂-selective adrenergic agonists such as apraclonidine and the like; Carbonic Anhydrase Inhibitors including such as acetazolamide, dichlorphenamide, methazolamide, brinzolamide, dorzolamide; Cholinergic Agonists such as carbachol, pilocarpine hydrochloride, pilocarpine nitrate, pilocarpine; Cholinesterase inhibitors such as demecarium, echothiophate, physostigmine.

Another embodiment of the invention relates to a composition comprising a compound of formula (I) and at least another active agent selected from the following classes of drugs: Beta-adrenergic antagonists including carteolol, levobunolol, metipranolol, timolol hemihydrate; Adrenergic Agonists including non-selective adrenergic agonists such as epinephrine borate, epinephrine hydrochloride, and dipivefrin; and Alpha₂-selective adrenergic agonists such as apraclonidine and the like; Carbonic Anhydrase Inhibitors including such as acetazolamide, dichlorphenamide, methazolamide, brinzolamide, dorzolamide; Cholinergic Agonists such as carbachol, pilocarpine hydrochloride, pilocarpine nitrate, pilocarpine; Cholinesterase inhibitors such as demecarium, echothiophate, physostigmine.

### General synthesis

The compound of formula (I) wherein R is selected from:
A1): -CH₂-(CHR¹)-NH-(C=O)-(CH₂)ₘ-[O-(CH₂)ₙ]ₚ-(CH-ONO₂)_{q}-CH₂-ONO₂
A2): -CH₂-(CHR¹)-NH-(C=O)-O-(CH₂)ₘ₁-[O-(CH₂)ₙ]ₚ-(CH-ONO₂)_{q}-CH₂-ONO₂
A3): 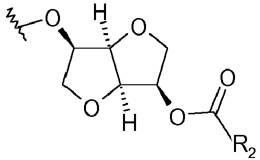 wherein:
R¹, R₂, m, m1, n, p and q are as above defined;
can be prepared:
- by reacting a compound of formula (IIa) 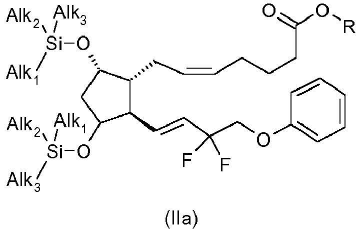 wherein R is as defined above and Alk₁, Alk₂ and Alk₃ are independently a C₁-C₄ straight or branched alkyl chain, with a fluoride ion source selected from tetra-n-butylammonium fluoride (TBAF), triethylamine trihydrofluoride (TREAT.HF) or hydrogen fluoride pyridine (pyridine.HF), in a suitable solvent such as THF, at temperature ranging from 0°C and 40°C as depicted in the below reported Scheme 1; 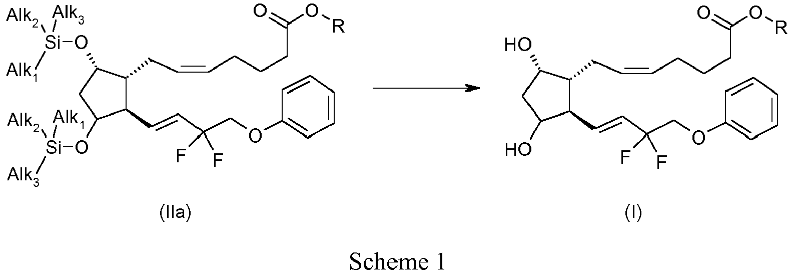
- or by reacting a compound of formula (IIb) 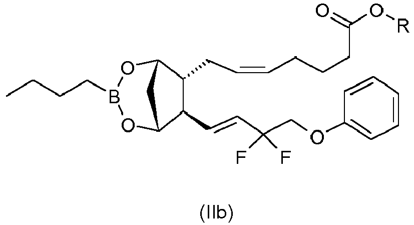 wherein R is as defined above with methanol or other alcohols as depicted in the below reported scheme 2;

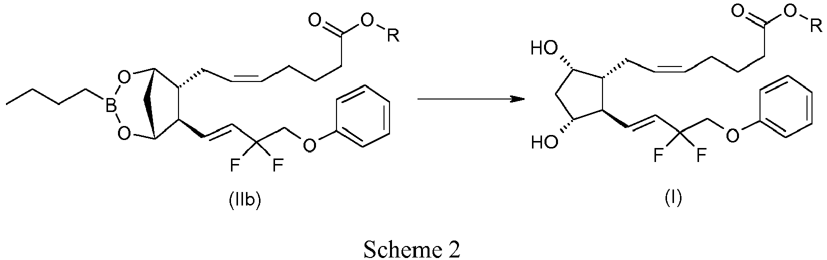

Compounds of formula (IIa) wherein R, Alk₁, Alk₂ and Alk₃, are as defined above and (IIb), wherein R is as above defined, can be generally prepared by reacting a compound of formula (IIIa) or (IIIb) wherein R, Alk₁, Alk₂ and Alk₃, are as above defined, with compounds of formula (IVa-c):

(IVa) = HO-CH₂-(CHR¹)-NH-(C=O)-(CH₂)ₘ-[O-(CH₂)ₙ]ₚ-(CH-ONO₂)_{q}-CH₂-ONO₂

(IVb) = HO-CH₂-(CHR¹)-NH-(C=O)-O-(CH₂)ₘ₁-[O-(CH₂)ₙ]ₚ-(CH-ONO₂)_{q}-CH₂-ONO₂

(IVc) = 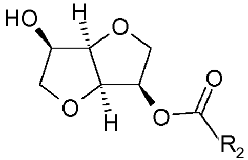 wherein R¹, R², m, m1, n, p and q are as above defined, as depicted in the below reported schemes 3a and 3b: 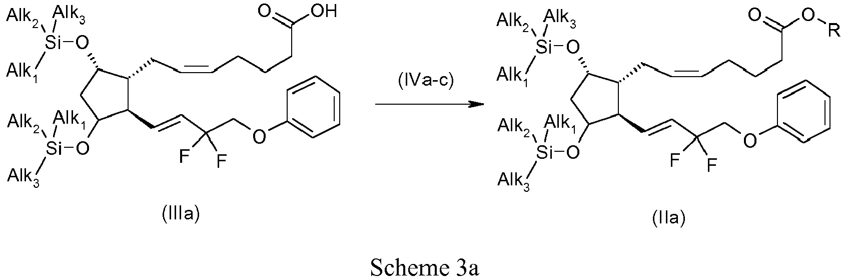 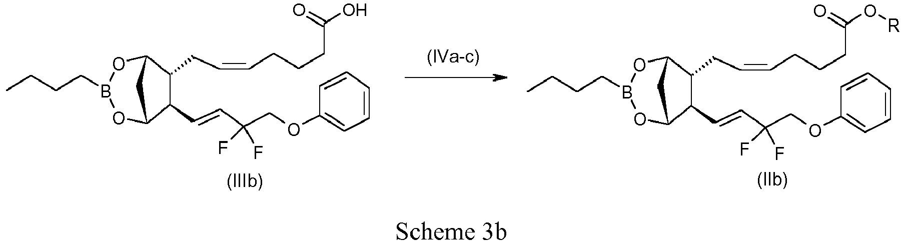 the reactions are carried out in a aprotic polar/non polar solvent such as THF, DMF or CH₂Cl₂, in presence of a coupling agent such as N,N'-dicyclohexylcarbodiimide (DCC), 1-ethyl-3-(3-dimethylaminopropyl)carbodiimide hydrochloride (EDC), O-(benzotriazol-1-yl)-N,N,N',N'-tetramethyluronium hexafluorophosphate (HBTU) or 1-[bis(dimethylamino)methylene]-1H-1,2,3-triazolo[4,5-b]pyridinium 3-oxid hexafluorophosphate (HATU), in presence of catalytic amount of DMAP at temperature ranging from -0°C to 80°C.

The compound of formula (IIIa) can be generally prepared from tafluprost free acid and silylating the hydroxyl groups by known methods such as for example by reacting tafluprost free acid with t-But(Me)₂-SiCl using procedure similar to those described in the literature (see for example: T.W. Greene, P. G. M. Wuts "Protective groups in organic Synthesis", 4th edition, J. Wiley & Sons, New York, 2006).
The compound of formula (IIIb) can be generally prepared by reacting tafluprost free acid with butylboronic acid following a general procedure reported in Organic Syntheses, Coll. Vol. 10, p.613 (2004).
Compounds of formula (IVa) can be generally prepared by reacting commercially available compounds of formula (V)

(V) = HO-CH₂-(CHR¹)-NH₂

wherein R¹ is H or CH₃, with compounds of formula (VI)
(VI) = HOOC-(CH₂)ₘ-[O-(CH₂)ₙ]ₚ-(CH-ONO₂)_{q}-CH₂-ONO₂
wherein m, n, p, q are as above defined; the reaction is carried out in a aprotic polar/non polar solvent such as THF, DMF or CH₂Cl₂, in presence of a coupling agent such as N,N'-dicyclohexylcarbodiimide (DCC), 1-ethyl-3-(3-dimethylaminopropyl)carbodiimide hydrochloride (EDC), O-(benzotriazol-1-yl)-N,N,N',N'-tetramethyluronium hexafluorophosphate (HBTU) or 1-[bis(dimethylamino)methylene]-1H-1,2,3-triazolo[4,5-b]pyridinium 3-oxid hexafluorophosphate (HATU), in presence of catalytic amount of DMAP at temperature ranging from -0°C to 80°C.

Alternatively, the compounds of formula (IVa) can be prepared by reacting a compound of formula (V) with compounds of formula (VIIa):

(VIIa) = XOC-(CH₂)ₘ-[O-(CH₂)ₙ]ₚ-(CH-ONO₂)_{q}-CH₂-ONO₂

wherein X is -Cl, p-nitrophenoxy, pentafluorophenoxy or 2,5-pyrrolidinedione, 1-oxy;
when X = -Cl the reaction is carried out in presence of a base such as DMAP, pyridine or triethylamine or K₂CO₃, Cs₂CO₃ in an aprotic/non polar solvent such as THF, DMF or CH₂Cl₂, at a temperature ranging from -20°C to 60°C.

When X is selected from p-nitrophenoxy, pentafluorophenoxy or 2,5-pyrrolidinedione, 1-oxy, the reaction is carried out in a aprotic polar/non polar solvent such as THF, DMF or CH₂Cl₂, in presence of DMAP, with or without a catalyst such as scandium triflate at temperature ranging from 0°C to 80°C.

The compounds of formula (IVb) can be prepared by reacting a compound of formula (V) with compounds of formula (VIIb):

(VIIb) = X¹CO-O-(CH₂)ₘ₁-[O-(CH₂)ₙ]ₚ-(CH-ONO₂)_{q}-CH₂-ONO₂

wherein X¹ is p-nitrophenoxy, pentafluorophenoxy or 2,5-pyrrolidinedione, 1-oxy; the reaction is carried out in a aprotic polar/non polar solvent such as THF, DMF or CH₂Cl₂, in presence of DMAP, with or without a catalyst such as scandium triflate at temperature ranging from 0°C to 80°C.

Compound (VI) are known in the art or can be prepared from known compounds by known methods, for example compounds (VI) can be prepared from the corresponding alcohols of formula (VIIIa)

(VIIIa) = HO-(CH₂)ₘ₁-[O-(CH₂)ₙ]ₚ-(CH-ONO₂)_{q}-CH₂-ONO₂

wherein m1, n, p and q are as above defined, by oxidation with known agents such as 2,2,6,6-Tetramethylpiperidinyloxy (TEMPO).

Compound (VIIa) wherein X is as previously defined can be prepared from compound (VI) by known methods.

Compound (VIIb), wherein X¹ is as previously defined ,can be generally prepared from compounds of formula (VIIIa) above defined by methods known in the art.

Compounds (IVc) can be prepared with reference to the preparation examples described in WO2009/098113 or can be prepared by reacting (3R,3aR,6R,6aR)-6-(3,4-dihydro-2H-pyran-6-yloxy)hexahydrofuro[3,2-b]furan-3-ol with compounds (VI) or (VIIa) as above described for compound (III).

## Claims

1. An compound of formula (I) 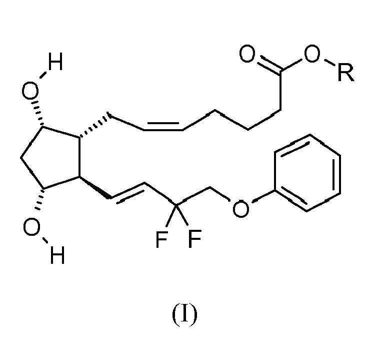 wherein
R is selected from
A1): -CH₂-(CHR¹)-NH-(C=O)-(CH₂)ₘ-[O-(CH₂)ₙ]ₚ-(CH-ONO₂)_{q}-CH₂-ONO₂
A2): -CH₂-(CHR¹)-NH-(C=O)-O-(CH₂)ₘ₁-[O-(CH₂)ₙ]ₚ-(CH-ONO₂)_{q}-CH₂-ONO₂
A3): 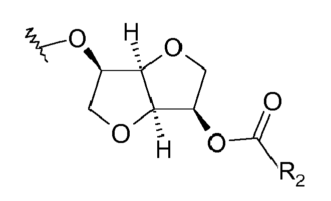 wherein
R¹ is -H or -CH₃;
R₂ is -(CH₂)ₘ-[O-(CH₂)ₙ]ₚ-(CH-ONO₂)_{q}-CH₂-ONO₂
p is 1 or 0,
q is 1 or 0,
m is an integer ranging from 1 to 10;
m1 is an integer ranging from 2 to 10;
n is an integer ranging from 1 to 6.

2. A compound of formula (I) according to claim 1 wherein
R is A1): -CH₂-(CHR¹)-NH-(C=O)-(CH₂)ₘ-[O-(CH₂)ₙ]ₚ-(CH-ONO₂)_{q}-CH₂-ONO₂
wherein
R₁ is -H or -CH₃;
p is 1 or 0,
q is 1 or 0,
m is an integer ranging from 1 to 6;
n is an integer ranging from 1 to 6.

3. A compound according to claim 2 wherein R is selected from the following group of linkers having structure A1:

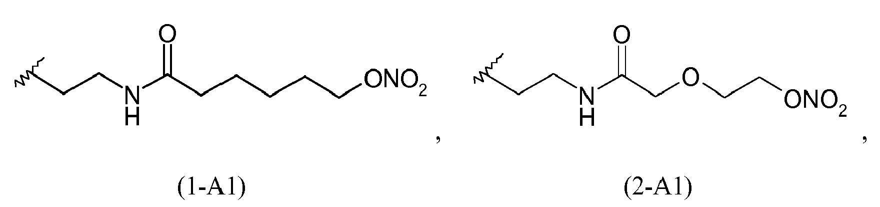

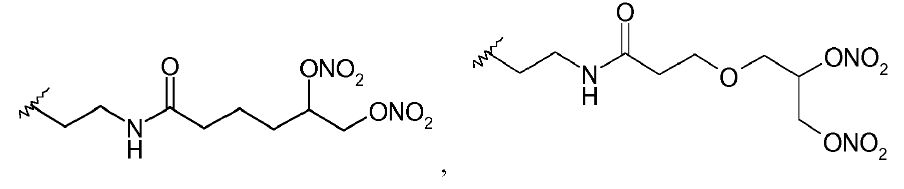

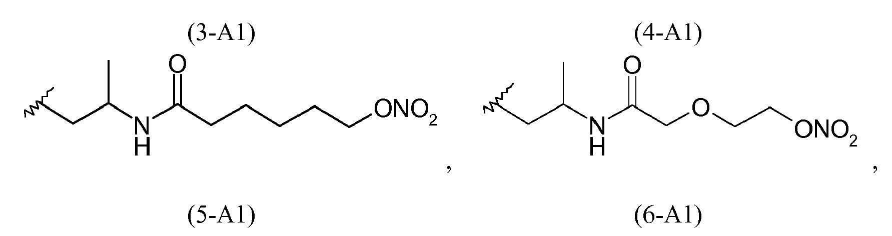

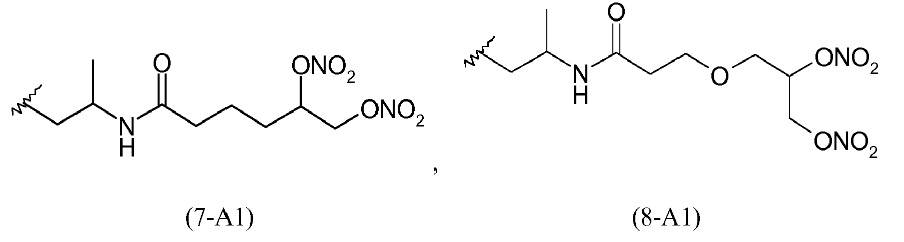

4. A compound of formula (I) according to claim 1 wherein
R is
A2): -CH₂-(CHR¹)-NH-(C=O)-O-(CH₂)ₘ₁-[O-(CH₂)ₙ]ₚ-(CH-ONO₂)_{q}-CH₂-ONO₂ R¹ is -H or -CH₃;
p is 1 or 0,
q is 1 or 0,
m1 is an integer ranging from 2 to 6;
n is an integer ranging from 1 to 6.

5. A compound according to claim 4 wherein R is selected from the following group of linkers having structure A2:

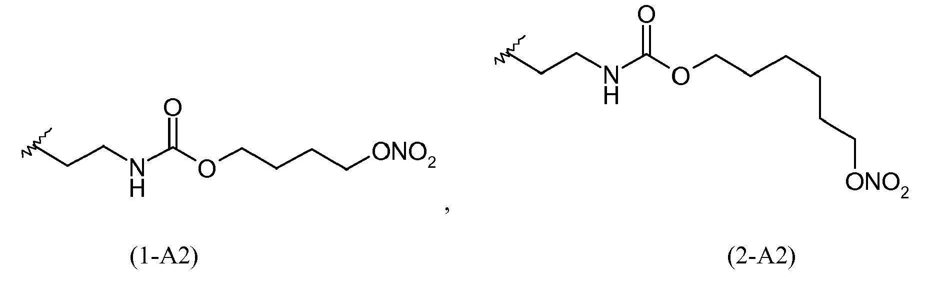

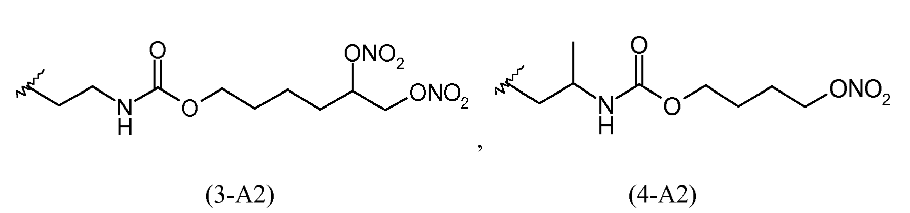

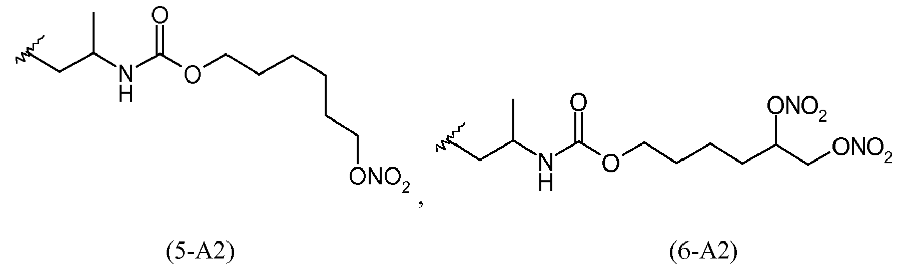

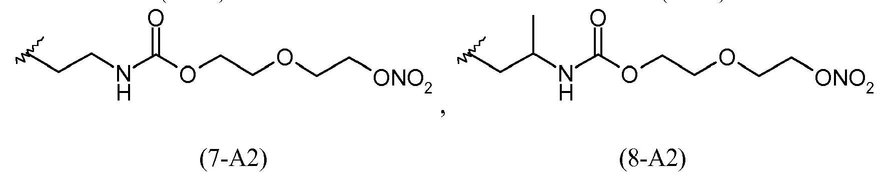

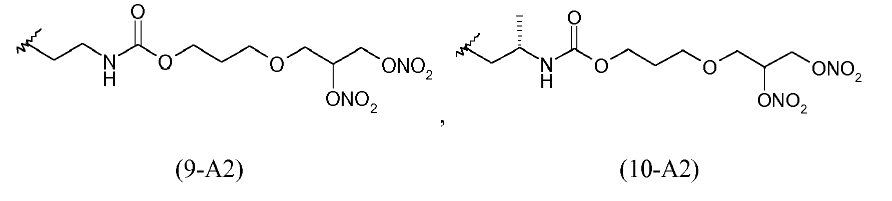

6. A compound of formula (I) according to wherein R is A3): 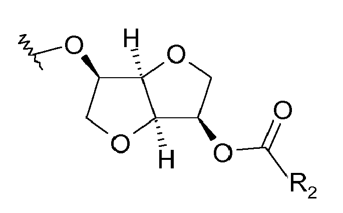 wherein
R₂ is -(CH₂)ₘ-[O-(CH₂)ₙ]ₚ-(CH-ONO₂)_{q}-CH₂-ONO₂
p is 1 or 0,
q is 1 or 0,
m is an integer ranging from 1 to 6;
n is an integer ranging from 1 to 6.

7. A compound according to claim 6 wherein R is selected from the following group of linkers having structure A3:

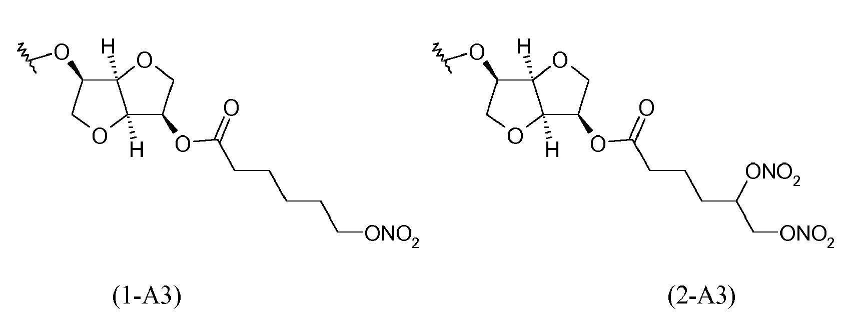

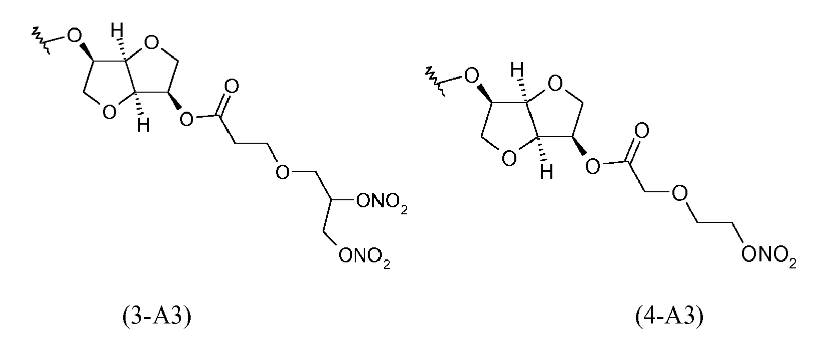

8. A compound of formula (I) according to claim 1 selected from the following group of compounds:
(Z)-2-(6-(nitrooxy)hexanamido)ethyl 7-((1R,2R,3R,5S)-2-((E)-3,3-difluoro-4-phenoxybut-1-enyl)-3,5-dihydroxycyclopentyl)hept-5-enoate (Compound (1));
(Z)-2-(5,6-bis(nitrooxy)hexanamido)ethyl 7-((1R,2R,3R,5S)-2-((E)-3,3-difluoro-4-phenoxybut-1-enyl)-3,5-dihydroxycyclopentyl)hept-5-enoate (Compound (2));
(Z)-2-(2-(2-(nitrooxy)ethoxy)acetamido)ethyl 7-((1R,2R,3R,5S)-2-((E)-3,3-difluoro-4-phenoxybut-1-enyl)-3,5-dihydroxycyclopentyl)hept-5-enoate (Compound (3));
(Z)-2-(3-(2,3-bis(nitrooxy)propoxy)propanamido)ethyl 7-((1R,2R,3R,5S)-2-((E)-3,3-difluoro-4-phenoxybut-1-enyl)-3,5-dihydroxycyclopentyl)hept-5-enoate (Compound (4));
(Z)-((S)-2-(6-(nitrooxy)hexanamido)propyl) 7-((1R,2R,3R,5S)-2-((E)-3,3-difluoro-4-phenoxybut-1-enyl)-3,5-dihydroxycyclopentyl)hept-5-enoate (Compound (5));
(Z)-((2S)-2-(5,6-bis(nitrooxy)hexanamido)propyl) 7-((1R,2R,3R,5S)-2-((E)-3,3-difluoro-4-phenoxybut-1-enyl)-3,5-dihydroxycyclopentyl)hept-5-enoate (Compound (6));
(Z)-((S)-2-(2-(2-(nitrooxy)ethoxy)acetamido)propyl) 7-((1R,2R,3R,5S)-2-((E)-3,3-difluoro-4-phenoxybut-1-enyl)-3,5-dihydroxycyclopentyl)hept-5-enoate (compound (7));
(Z)-((2S)-2-(3-(2,3-bis(nitrooxy)propoxy)propanamido)propyl) 7-((1R,2R,3R,5S)-2-((E)-3,3-difluoro-4-phenoxybut-1-enyl)-3,5-dihydroxycyclopentyl)hept-5-enoate (Compound (8));
(Z)-2-((4-(nitrooxy)butoxy)carbonylamino)ethyl 7-((1R,2R,3R,5S)-2-((E)-3,3-difluoro-4-phenoxybut-1-enyl)-3,5-dihydroxycyclopentyl)hept-5-enoate (Compound (9));
(Z)-((S)-2-((4-(nitrooxy)butoxy)carbonylamino)propyl) 7-((1R,2R,3R,5S)-2-((E)-3,3-difluoro-4-phenoxybut-1-enyl)-3,5-dihydroxycyclopentyl)hept-5-enoate (Compound (10));
(Z)-2-((6-(nitrooxy)hexyloxy)carbonylamino)ethyl 7-((1R,2R,3R,5S)-2-((E)-3,3-difluoro-4-phenoxybut-1-enyl)-3,5-dihydroxycyclopentyl)hept-5-enoate (Compound (11));
(Z)-((S)-2-((6-(nitrooxy)hexyloxy)carbonylamino)propyl) 7-((1R,2R,3R,5S)-2-((E)-3,3-difluoro-4-phenoxybut-1-enyl)-3,5-dihydroxycyclopentyl)hept-5-enoate (Compound (12));
(Z)-2-((5,6-bis(nitrooxy)hexyloxy)carbonylamino)ethyl 7-((1R,2R,3R,5S)-2-((E)-3,3-difluoro-4-phenoxybut-1-enyl)-3,5-dihydroxycyclopentyl)hept-5-enoate (Compound (13));
(Z)-((2S)-2-((5,6-bis(nitrooxy)hexyloxy)carbonylamino)propyl) 7-((1R,2R,3R,5S)-2-((E)-3,3-difluoro-4-phenoxybut-1-enyl)-3,5-dihydroxycyclopentyl)hept-5-enoate (Compound (14));
(Z)-2-((2-(2-(nitrooxy)ethoxy)ethoxy)carbonylamino)ethyl 7-((1R,2R,3R,5S)-2-((E)-3,3-difluoro-4-phenoxybut-1-enyl)-3,5-dihydroxycyclopentyl)hept-5-enoate (Compound (15));
(Z)-((S)-2-((2-(2-(nitrooxy)ethoxy)ethoxy)carbonylamino)propyl) 7-((1R,2R,3R,5S) -2-((E)-3,3-difluoro-4-phenoxybut-1-enyl)-3,5-dihydroxycyclopentyl)hept-5-enoate (Compound (16));
(Z)-2-((3-(2,3-bis(nitrooxy)propoxy)propoxy)carbonylamino)ethyl 7-((1R,2R,3R, 5S)-2-((E)-3,3-difluoro-4-phenoxybut-1-enyl)-3,5-dihydroxycyclopentyl)hept-5-enoate (Compound (17));
(Z)-((2S)-2-((3-(2,3-bis(nitrooxy)propoxy)propoxy)carbonylamino)propyl) 7-((1R,2R,3R,5S)-2-((E)-3,3-difluoro-4-phenoxybut-1-enyl)-3,5-dihydroxycyclopentyl) hept-5-enoate (Compound (18));
(Z)-((3R,3aR,6R,6aR)-6-(6-(nitrooxy)hexanoyloxy)hexahydrofuro[3,2-b]furan-3-yl) 7-((1R,2R,3R,5S)-2-((E)-3,3-difluoro-4-phenoxybut-1-enyl)-3,5-dihydroxycyclo pentyl)hept-5-enoate (Compound (19));
(Z)-((3R,3aR,6R,6aR)-6-(5,6-bis(nitrooxy)hexanoyloxy)hexahydrofuro[3,2-b]furan-3-yl) 7-((1R,2R,3R,5S)-2-((E)-3,3-difluoro-4-phenoxybut-1-enyl)-3,5-dihydroxy cyclopentyl) hept-5-enoate (Compound (20));
(Z)-((3R,3aR,6R,6aR)-6-(2-(2-(nitrooxy)ethoxy)acetoxy)hexahydrofuro[3,2-b]furan-3-yl) 7-((1R,2R,3R,5S)-2-((E)-3,3-difluoro-4-phenoxybut-1-enyl)-3,5-dihydroxy cyclopentyl)hept-5-enoate (Compound (21));
(Z)-((3R,3aR,6R,6aR)-6-(3-(2,3bis(nitrooxy)propoxy)propanoyloxy)hexahydrofuro[3,2-b]furan-3-yl) 7-((1R,2R,3R,5S)-2-((E)-3,3-difluoro-4-phenoxybut-1-enyl)-3,5-dihydroxycyclopentyl)hept-5-enoate (Compound (22)).

9. A compound of formula (I) according to any of claims 1 to 8 for use as medicament.

10. A compound of formula (I) according to any of claims 1 to 8 for use in the treatment of ocular hypertension.

11. A compound of formula (I) according to any of claims 1 to 8 for use in the treatment of ocular hypertension or glaucoma.

12. A compound of formula (I) for the use according to claim 11, wherein glaucoma is primary open angle glaucoma, normal intraocular tension glaucoma, pseudoexfoliation glaucoma, acute angle-closure glaucoma or chronic closed angle glaucoma.

13. A topical ocular pharmaceutical composition comprising a compound of formula (I) according to any of claims 1 to 8 as active principle and a pharmaceutically acceptable excipient or a combination of excipients.

14. A composition comprising a compound of formula (I) according to any of claims 1 to 8 and at least another active agent selected from the following classes of drugs: Beta-adrenergic antagonists, Adrenergic agonists, Alpha₂-selective adrenergic agonists, Carbonic Anhydrase Inhibitors, Cholinergic agonists, Cholinesterase inhibitors.

15. A composition according to claim 14 for use in the treatment of ocular hypertension or glaucoma.
